# EUROPEAN PATENT SPECIFICATION

(11) **EP 3 914 350 B1**
(45) Date of publication and mention of the grant of the patent: **15.03.2023**
(21) Application number: 20845449.6
(22) Date of filing: 28.12.2020
(51) Int. Cl.: A61P 17/02, A61K 33/06, A61K 36/185, A61K 36/328, A61K 36/886

(54) **UNIQUE HERBAL COMBINATION FOR THE TREATMENT OF ANAL FISSURES AND WOUNDS IN PHARMACEUTICAL DOSAGE FORM**
EINZIGARTIGE KRÄUTERKOMBINATION ZUR BEHANDLUNG VON ANALFISSUREN UND WUNDEN IN PHARMAZEUTISCHER DOSIERUNGSFORM
COMBINAISON UNIQUE DE PLANTES LE TRAITEMENT DE FISSURES ET DE PLAIES ANALES SOUS UNE FORME POSOLOGIQUE PHARMACEUTIQUE

(30) Priority: 01.01.2020 GC 202038941
(43) Date of publication of application: 01.12.2021
(73) Proprietor: Saudi Pharmaceutical Industries & Medical Appliances Corporation Spimaco Addwaeih, Alqassim (SA)
(72) Inventor: ALANAZI, Fars Kaed M, Riyadh, 14213 (SA)
(74) Representative: Ilievski, Bogoljub
(86) International application number: PCT/SA2020/050019
(87) International publication number: WO 2021/137743

(56) References cited:
- EP-A1- 0 923 935
- CN-A- 1 328 836
- CN-A- 105 232 447
- CN-A- 105 560 367
- CN-A- 112 057 568
- FR-A1- 2 152 408
- FR-A1- 2 768 342
- SA-B- 4 550
- N Rahmani ET AL: "Effects of Aloe vera cream on chronic anal fissure pain, wound healing and hemorrhaging upon defection: a prospective double blind clinical trial", European Review for Medical and Pharmacological Sciences, 1 January 2014 (2014-01-01), pages 1078-1084, XP055387806, Retrieved from the Internet: URL:http://www.europeanreview.org/wp/wp-co ntent/uploads/1078-1084.pdf [retrieved on 2017-07-04] cited in the application
- CHIDAMBARA MURTHY K.N. ET AL: "Study on Wound Healing Activity of Punica granatum Peel", JOURNAL OF MEDICINAL FOOD, vol. 7, no. 2, 1 June 2004 (2004-06-01), pages 256-259, XP055784086, US ISSN: 1096-620X, DOI: 10.1089/1096620041224111 Retrieved from the Internet: URL:http://dx.doi.org/10.1089/109662004122 4111>

## Description

### Background of the invention

Anal wounds are a common painful disorder of the anal area that are characterized by un-tolerated pain upon defecation, anal bleeding, and anal sphincter spasms (1). Anal wounds are usually caused by trauma or injury to the anal canal while passing hard or large stools, constipation, diarrhea or childbirth (2). It could end with difficulty with bowel movements with severe pain and discomfort; till leading to major complications like clotting.

Treatment usually includes adopting simple methods to have stool soft such as increasing fiber and fluid intake to roll out the reason behind the formation of new wounds. Traditional practice such as soaking in warm water for 10-20 minutes as often as possible, particularly after bowel movements, also helps with healing and reducing discomfort. If symptoms still persist, further treatment is required which involves initially using special cream based. Topical anesthetics and pain medication may also be prescribed to control pain. Sometimes, injection of botulin toxin into the internal anal sphincter could be used as alternative treatment (3).

However, some patients with acute conditions do not heal and become chronic wounds. Surgery is recommended if the symptoms do not respond to conservative treatment. Anal surgery is aimed to re-establish the conditions which lead to healing of the small mucosa tears. This includes either a partial division of the internal sphincter (sphincterotomy) or manual dilatation of the anus. Surgical treatment for this condition has been associated with the side-effect and incontinence in up to 30% of patients (4). Therefore, a non-surgical method for the treatment of chronic anal fissures is highly desirable.

Over the past few years, a number of clinical studies have shown that topical application of ointments promotes the healing of chronic anal wounds (5). The important of development depends on the composition of the healing ingredients since some of them have complicated side effects (6).

Wound healing is a complex multifactorial process that results in the contraction and closure of the wound and restoration of a functional barrier (35). Repair of injured tissues occurs as a sequence of events, which includes inflammation, proliferation and migration of different cell types The difficulties in treatment becomes more challenges especially in case of possibility of wound become infected by fecal bacteria.

Aloe vera (L.) Burm. f. belongs to the Xanthorrhoeaceae plant family and has long been used as a traditional medicine. It is one of the most recognizable herbs in the world and the medicinal part of this plant is the succulent leaf. Aloe vera juice is obtained by breaking or slicing a leaf and is the principal part of the plant that is used in herbal medicine. It also has pharmacological properties that have been shown to provide antioxidant, wound healing, antibacterial, antifungal, and immunomodulatory effects (7). Recently, it has been shown that Aloe vera cream can facilitate wound healing in post hemorrhoidectomy patients (8). In addition, another study demonstrated that an aloe cream reduced the healing time in patients with burn injuries compared to silver cream (9).

Pomegranate is one of the important fruits stated in Qur'an. *Punica granatum* belongs to the family of Punicaceae, is commonly known as pomegranate, grenade, granats and punica apple (10). Pomegranate extract are becoming popular in the Western world for the treatment and prevention of arthritis and other inflammatory diseases (11). Pomegranate peels have been used since antiquity in the Middle East as colorant for textiles because of their high tannin and phenolic contents (12). Also, among the peel, pulp and seed pomegranate peel had the highest antioxidant activity (13, 14-17). In many culture's folk medicine pomegranates have been used extensively (18). Pomegranate peel contains large amounts of polyphenols such as ellagic tannins, ellagic acid and gallic acid. It has been used in the preparation of tinctures, cosmetics, therapeutic formulae and food recipes (19) Dried fruit peel is used for diarrhea and to treat respiratory and urinary tract infections (20). Anti- fertility effect (21) cytotoxic activity (22,23) hepatoprotective activity (24) and hypoglycemic activity (25). MURTHY et al., (26) studied the wound healing activity of phenol-rich methanolic extract of dried pomegranate peel on the skin of Wistar rats. Following the application of the extract, formulated as a water-soluble gel, the animals treated with 5% gel showed complete healing after 10 days.

Myrrh, is the stem resinous exudates belonging to different Commiphora species according to their growing area. It is an oleo-gum resin that form emulsion upon mixing with water. Published researches showed different medicinal effects for myrrh. The medical uses including fasciolicidal effect, and in the treatment of schistosomiasis (27-29). Myrrh as a whole or some of its components appear to have many therapeutic effectiveness in the treatment of different diseases as treating gynecologic cancer disease (30), anaesthetic, antibacterial, antifungal and antihyperglycemic (31,32). Other medicinal use include antiulcer, antipyretic, analgesic, antioxidant and anti-inflammatory (31, 33). Myrrh contained about 2-8% essential oil, 40-60% water-soluble gum and 23-40% alcohol-soluble resins. The essential oil was rich in furanosesquiterpenoids where about 20 compound have been isolated (34).

Many scientific reports were published for single use of the invention four ingredients for wound healing (7-34). Three combination of herbal ingredients were also presented by Ehab at el., (35). None of the prior art documents has provided the synergistic effect of four ingredient working for the treatment of anal wounds unexpectedly and for the first time.

### Summary of the invention

The present invention relates to an integrated herbal pharmaceutical composition for producing a synergistic effect for the treatment of anal wounds.
The said composition comprises an ethanolic extract of pomegranate peel, aqueous extract of myrrh and alum (potassium aluminum sulfate), and a pharmaceutical carrier.
The composition may further comprises Aloe Vera oil.
In a preferred embodiment, the said components are present in a range of 5-20% wt/wt.
In a further embodiment, the composition also comprises ≤ 5% w/w Aloe Vera oil wt/wt.

The present invention is considered innovative because it unexpectedly provides a synergistic effect of the ingredients of said composition, working in combination for the treatment of anal wounds for the first time. In the closest prior art, Saudi patent 4550, it was disclosed that the components comprise different extracts. It is well known for a person skilled in the art that ethanolic and aqueous extraction procedures result in different extracts. In the current invention, ethanolic pomegranate peel extract was used while in 4550, aqueous pomegranate peel extract was used. Oppositely also, in the current invention, aqueous extract for myrrh was used while in the 4550, ethanolic extract from myrrh was used. Also, the additional use of Aloe Vera oil in the current invention provides for a four-component mixture which results in an increased wound healing as compared to Aloe Vera oil alone or the three-component mixture described in 4550 or herein.

### Brief description of the drawings:

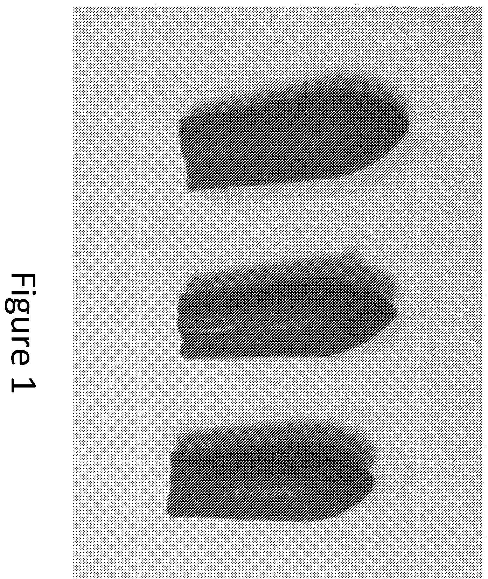

**Figure 1****:** Pictures of the prepared suppositories.

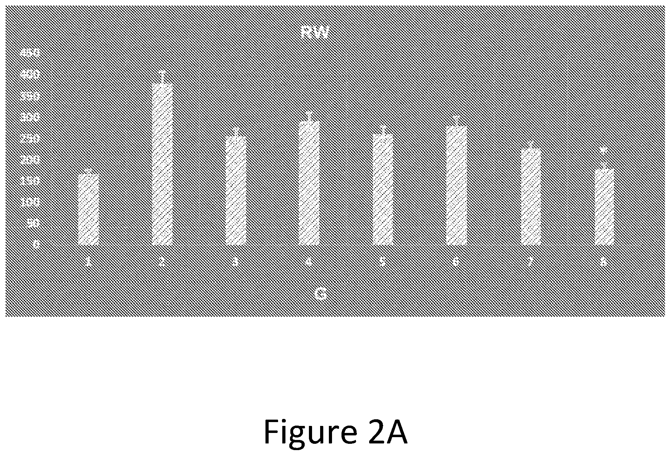

**Figure 2A****:** The rectum weight data for : Group 1 (negative control); Group 2 ( positive control ), Group 3 (administered suppository containing only Pomegranate); Group 4 (administered suppository containing only Myrrh), Group 5 (administered suppository containing only Alum), Group 6 (administered suppository containing only Aloe vera oil), Group 7 (administered the prepared suppositories containing: Pomegranate + Myrrh+ Alum), Group 8 (administered the prepared suppositories containing: Pomegranate + Myrrh+ Alum + Aloe vera oil). * presented statistically significant from others treatments; (p< 0.05).

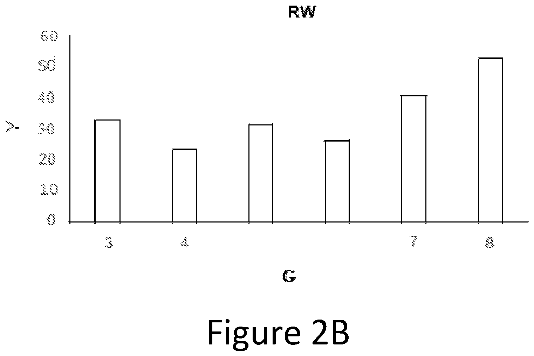

**Figure 2B****:** The percentage reduction in rectum weight data for Group 3 (administered suppository containing only Pomegranate); Group 4 (administered suppository containing only Myrrh), Group 5 (administered suppository containing only Alum), Group 6 (administered suppository containing only Aloe vera oil), Group 7 (administered the prepared suppositories containing: Pomegranate + Myrrh+ Alum), Group 8 (administered the prepared suppositories containing: Pomegranate + Myrrh+ Alum + Aloe vera oil).

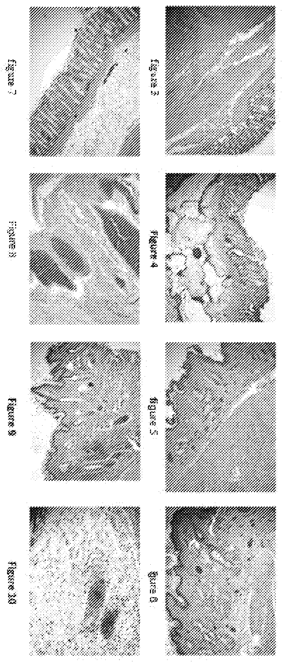

**Figures 3-10****:** show images of rectal (anus) tissues for wistar rats as follows: Figure 3: Negative control (normal animals); Figure 4: Positive Control (untreated wounds); Figure 5: Pomegranate suppository treated animals; Figure 6: Myrrh suppository base treated animals; Figure 7: Alum suppository treated animals; Figure 8: Aloe Vera oil suppository treated animals; Figure 9: Alum+ Pomegranate + Myrrh suppository treated animals; and Figure 10: Alum+ Pomegranate + Myrrh+ Aloe Vera oil suppository treated animals

### Detailed description

### Methodology:

The herbal extracts were purchased from GMP supplier (Table 1). All chemical used are of analytical or pharmaceutical grade.

**Table 1: Source of herbal and medicated substances.**

| | Material | Supplier |
|---|---|---|
| 1 | Dried Ethanolic pomegranate peel extract | LDA International, China |
| 2 | Dried Aqueous myrrh extract | LDA International, China |
| 3 | ALUMINIUM POTASSIUM SULFATE DODECAHYDRATE | Sigma-Aldrich, USA |
| 4 | Aloe vera oil | LDA International, China |

### Specification of Prepared Suppositories:

The following table shows the pharmaceutical specification of the prepared suppositories.

**Table 2: the prepared suppositories pharmaceutical specification**

| **Parameters** | **Specification** |
|---|---|
| **Description** | Brown to dark brown, torpedo shaped suppositories. |
| **Identification of Pomegranate Ellagic Acid (HPLC)** | Complies |
| **Identification of Aluminum Potassium Sulfate Aluminum (ICP)** | Complies |
| **Identification of Myrrh (TLC)** | Complies |
| **Uniformity of Weight** | Complies with BP |
| **Disintegration Time (in Purified Water)** | 30 min |
| **Hardness** | Complies |
| **Microbial Examination Test Total Aerobic Microbial Count Total Combined Yeast and Moulds Count** | Complies |

### Pharmaceutical Composition

The pharmaceutical composition intended for use as a medicament for the treatment of anal wounds, both internal and external use, comprises:
(a) 5-20% w/w of ethanolic pomegranate peel extract,
(b) 5- 20% w/w of aqueous myrrh extract,
(c) 5-20% w/w alum, and
(d) a pharmaceutical carrier.

In a preferred embodiment of the current invention, the components are in the following ranges:
(a) 10-15% w/w of ethanolic pomegranate peel extract,
(b) 10-15% w/w of aqueous myrrh extract,
(c) 10-15% w/w alum, and
(d) a pharmaceutical carrier.

In a further preferred embodiment, the ethanolic pomegranate peel extract is present is greater than 10% w/w, the aqueous myrrh extract is greater than 10% w/w, the alum is greater than 10% w/w and the Aloe Vera oil is lower than 10% w/w.

In yet a further embodiment, the pharmaceutical composition comprises between 1-20% w/w Aloe Vera oil, preferably between 1-5% w/w Aloe Vera oil.

### Induction of anal wounds:

Hemorrhoids were induced in male wistar rats by a modification of the methods described by Nishiki et al. (31) and Okumura et al.(32). In brief: male wistar rats (250±5g body weight) were used in this study. All rats were fasted from food for 3 days but allowed drinking water ad libitum. To induce hemorrhoids a mixture of 6% croton oil in diethyl ether: pyridine: diethyl ether: water in the ratio of 10: 4 : 5: 1 (v/v) was used.

On the day of experiment each rat was immobilized in restrainer cage. The tampon of an ear cotton bud (produced by septona SA, Greece and purchased from the local market in Riyadh, kingdom of Saudi Arabia) was dipped in 10 ml of the above inducer contained in a 10 ml vial for 60 seconds. Then it was inserted into the rectum and allowed to contact the mucosa to a depth of 2cm for 60 seconds and then removed smoothly. The animals were freed from their restrainers and placed in their cages with no food or water for complete 4 hours. The volume of the inducer absorbed by each tampon was 333 µl. The animals were then sacrificed by an overdose of diethyl ether. The lower abdomen was opened, all of the reproductive organs removed, the hip bone cut and the lower rectum cleared from any adhering tissues. Then a rectum-anus portion measuring 2 cm was cut from each rat starting from the circular hairline on the anus epithelium using a pair of campus and a pair of scissors. The cut piece was then opened longitudinally, blotted on a piece of filter paper and weighed immediately. The weight of tissues corresponding to 150 g body weight was then calculated in mg.

### Treatment of animals with the prepared suppositories

Male wistar rats (250±5g body weight) were fasted from food for 3 days but allowed drinking water ad libitum. The animals were divided into 3 groups:
Group 1(negative control) (not administered any suppository without wound inducing) but the cotton bud was immersed in distilled water only.
Group 2,( positive control) administered plain suppository.
Group 3, administered suppository containing only Pomegranate
Group 4, administered suppository containing only Myrrh
Group 5, administered suppository containing only Alum
Group 6, administered suppository containing only Aloe vera oil
Group 7, administered the prepared suppositories containing: Pomegranate + Myrrh+ Alum
Group 8, administered the prepared suppositories containing: Pomegranate + Myrrh+ Alum + Aloe vera oil (four ingredients' combination suppository)

Each suppository was allowed to contact the rectum for 1 hour. During this time, the anus was closed using a large bull-dog. Thereafter, the bull-dog was removed. The percentage reductions in the weights of recti of the treated animals were then calculated relative to the control weights (Table 3).

**Table 3: Rat rectum weight of normal and treated rats after simulation of hemorrhoid**

| | Rectum weight (mg) |
|---|---|
| (Group 1) | 166.35 ± 18.26 |
| (Group 2) | 380.81 ± 41.34 |
| (Group 3) | 255.41 ± 30.2 |
| (Group 4) | 291.16 ± 29.75 |
| (Group 5) | 260.41 ± 33.56 |
| (Group 6) | 280.24 ± 27.1 |
| (Group 7) | 225.17 ± 20.61 |
| (Group 8) | 180.12 ± 19.17 |

It is clear from the data that the invented formula has tremendously reduced the weight of the rectum which is statistically significant from other treatment (p< 0.05) (Table 3, Figure 2A and Figure 2B). The new invented formula with addition of Aloe Vera oil has more pronounced effect comparing to single treatment or the three combination. This because wound healing is complex process which needs more treatments modalities that may sometime synergies internal healing processes. For instance, Aloe vera preparations have many biological effects, including immunomodulatory, anti-inflammatory, antioxidant, and wound healing properties (36). These properties were synergized with Pomegranate, Myrrh and Alum.
It is evidently that figures 3-10 confirm previous results in rectum weight study. Normal mucosa and Normal blood vessels was shown with negative control group (Figure 3). Differently, in case of (Positive Control), it shows presence of edema in mucosa and submucousa, ulceration on surface, sever damage, extensive necrosis (Figure 4). In case of Pomegranate suppository, Skin appears normal with small Congested blood vessels meaning the need of improvement (Figure 5). Similarly, with Myrrh suppository Congested blood vessels and dilatation was existing (Figure 6). Due to the effect of Alum on blood vessels, Alum suppository had less necrosis in mucosa less hemorrhage (Figure 7). In case of Aloe Vera oil suppository, blood vessels still congested and dilated (Figure 8). The suppositories containing the three combination (Alum+ Pomegranate + Myrrh) showed that blood vessels still congested and dilated with no necrosis. To make the wound healing more alleviated, the synergistic action of the four ingredient was induced in (figure 10). Blood vessels completely healed and thick wall arterioles appears normal size.
List of symbols used in the figures:
G: groups
RW: rectum weight (mg)
Y: % reduction in mass (mg)

### References

1. N. RAHMANI 1, M. KHADEMLOO2, K. VOSOUGHI 1, S. ASSADPOUR3, Effects of Aloe vera cream on chronic anal fissure pain, wound healing and hemorrhaging upon defection: a prospective double blind clinical trial, European Review for Medical and Pharmacological Sciences, 2014; 18: 1078-1084
2. LOCK MR, THOMSON JPS. Fissure in ano: the initial management and prognosis. Br J Surg 1977; 64: 355-358.
**3.** Minkes RK1, Langer JC, A prospective study of botulinum toxin for internal anal sphincter hypertonicity in children with Hirschsprung's disease. J Pediatr Surg. 2000 Dec;35(12):1733-6.
4. NIELSEN MB, RASMUSSEN OO, PEDERSEN JF , CHRISTIANSEN J. Risk of sphincter damage and anal incontinence after anal dilatation for fissure in ano: An endosonographic study. Dis Colon Rectum 1993; 36:677-680.
5. LODER PB, NICHOLLS RJ, PHILLIPS RKS. Reversible chemical sphincterotomy by local appliation of gylceryl trinitrate.Br J Surg 1994;81:1386-1389.
6. LUND JN, SCHOLEFIELD JH. A randomised, prospective double-blind placebo-controlled trial of glyceryl trinitrate ointment in the treatment of anal fissure. Lancet 1997; 349:11-14.
7. HABEEB F , SHAKIR E, BRADBURY F , CAMERON P , TARAVATI MR, DRLTMMOND AJ, GRAY AI, FERRO VA. Screening methods used to determine the antimicrobial properties of Aloe vera inner gel. Methods 2007; 42:315-320.
8. ESHGHI F , HOSSEINIMEHR SJ, RAHMANI N, KHADEMLOO M, NOROZI MS, HOJATI O. Effects of aloe vera Cream on Posthemorrhoidectomy Pain and Wound Healing: Results of a Randomized, Blind, Placebo-Control Study.J Altern Complement Med 2010;6:647-650.
9. KHORASANI GA, HOSSEINIMEHR SJ, AZADBAKHT M, ZAMANI A, MAHDAVI MR. Aloe versus silver sulfadiazine creams for second-degree burns: a randomized controlled study. Surgery Today 2009; 7: 587-591.
10. Supayang P.V., Treechada S., Surasak L., Thanomjit S., Tetsuya I., Takeshi H., Inhibitory effects of active compounds from Punica granatum pericarp on verocytotoxin production by enterohemorrhagic Escherichia coli O157:H7. J. Health Sci. 51(5):590-596, (2005).
11. Ahmed S, Wang N, Hafeez BB, Cheruvu VK, Haqqi TM: Punica granatum L. extract inhibits IL-1beta-induced expression of matrix metalloproteinases by inhibiting the activation of MAP kinases and NF-kappa B in human chondrocytes in vitro. J Nutr, 135:2096-2102, (2005).
12. Kulkarni S.S., Gokhale A.V, Bodake U.M, Pathade G.R., Cotton Dyeing with Natural Dye Extracted from Pomegranate (Punica granatum) Peel, Universal Journal of Environmental Research and Technology, 1(2):135-139, (2011).
13. Guo, C. J., Yang, J. J., Wei, J. Y., Li, Y. F., Xu, J., & Jiang, Y. G. Antioxidant activities of peel, pulp and seed fractions of common fruits as determined by FRAP assay. Nutrition Research, 23, 1719-1726, (2003).
14. Abdel Moneim A.E., Antioxidant activities of Punica granatum (pomegranate) peel extract on brain of rats, Journal of Medicinal Plants Research, 6(2):195-199, (2012)
15. Li Y, Guo C, Yang J, Wei J, Xu J, Cheng S. Evaluation of antioxidant properties of pomegranate peel extract in comparison with pomegranate pulp extract. Food Chem, 2(96): 254-260, (2006).
16. Qu W, Pan Z, Zhang R, Ma H, Chen X, Atungulu GG. Integrated extraction and anaerobic digestion process for recovery of nutraceuticals and biogas from pomegranate marcs. Trans ASABE, 52(6): 1997-2006, (2009).
17. Qu W, Pan Z, Ma H. Extraction modeling and activities of antioxidants from pomegranate marc. J Food Eng, 99(1): 16-23, (2010) .
18. Longtin, R.. The pomegranate: nature's power fruit. Journal of National Cancer Institute, 95, 346-348, (2003).
19. Nasr, C. B., Ayed, N., & Metche, M.. Quantitative determination of the polyphenolic content of pomegranate peel. Z Lebensm Unters Forsch, 203, 374-378, (1996).
20. Yunfeng, L., G. Changjiang, J. Yang, J. Wei, J. Xu, and S. Cheng. Evaluation of antioxidant properties of pomegranate peel extract in comparison with pomegranate pulp extract. Food Chem. 96, 254-260, (2006).
21. Umadevi M. , Kumar P.K.S. , Bhowmik D. , Duraivel S., Medicinal Plants with Potential Antifertility Activity, Journal of Medicinal Plants Studies, 1(1): 26-33, (2013)
22. Ampasavate C., Okonogi S., Anuchapreeda S., Cytotoxicity of extracts from fruit plants against leukemic cell lines, Afr. J. Pharm. Pharmacol, 4(1): 13-21, (2010).
23. Abdul gany Z.S., Gedhan A.F., Hussen R.A., The Cytotoxic Activity of Punica granatum on Growth of Hela and REF Cell Lines, Iraqi Journal of Cancer and Medical Genetics, 3(1): 7-10, (2010).
24. Ashoush I.S., El-Batawy O.I., El-Shourbagy G.A., Antioxidant activity and hepatoprotective effect of pomegranate peel and whey powders in rats, Ann. Agric. Sci. (2013), http://dx.doi.org/10.1016/j.aoas.2013.01.005
25. Hontecillas R, O'Shea M, Einerhand A, Diguardo M, Bassaganya-Riera J. Activation of PPAR gamma and alpha by punicic acid ameliorates glucose tolerance and suppresses obesity-related inflammation. J Am Coll Nutr. 28(2): 184-95, (2009).
26. MURTHY K.N., REDDY V.K., VEIGAS J.M., MURTHY U.D.: Study on wound healing activity of Punica granatum peel. J. Med. Food., 2004, 7, 256-259.
27. El Ashry,E.S., Rashed,N., Salama,O.M., Saleh,A., Components, therapeutic value and uses of myrrh. Pharmazie 58,163-168, (2003).
28. Massoud, A.,Preliminary study of therapeutic efficacy of a new fasciolicidal drug derived from Commiphora molmol (myrrh). The American Journal of Tropical Medicine and Hygiene 65,96-99, (2001).
29. Abo-Madyan AA, Morsy TA, Motawea SM., Efficacy of Myrrh in the treatment of schistosomiasis (haematobium and mansoni) in Ezbet El-Bakly, Tamyia Center, El-Fayoum Governorate, Egypt., J Egypt Soc Parasitol.;34(2);423-446 (2004).
**30.** Su S., Wang T., Chen T., Duan J., Yu L., Tang Y., Cytotoxicity activity of extracts and compounds from Commiphora myrrha resin against human gynecologic cancer cells, J. Med. Plant. Res., 5(8), 1382-1389, (2011)
31. European Drugs Encyclopedia, Commiphora (Myrrha (Commiphora molmol)),Product Characteristics - Assessment Report, http://www.theodora.com/drugs/eu/myrrha_commiphora_molmol_herbal.html
32. Abdallah E.M., Khalid A.S., Ibrahim N., Antibacterial activity of oleo-gum resins of Commiphora molmol and Boswellia papyrifera against methicillin resistant Staphylococcus aureus (MRSA), Scientific Research and Essay, 4 (4); 351-356, (2009).
33. Shen T., HuiLi G., Wanga X., XiangLou H., The genus Commiphora: A review of its traditional uses, phytochemistry and pharmacology, Journal of Ethnopharmacology 142: 319-330, (2012).
34. Zhu, N.Q.,Sheng,S.Q.,Sang,S.M., Isolation and characterization of several aromatic sesquiterpenes from Commiphora myrrha. Flavour Fragrance Journal 18, 282-285, (2003).
35. Ehab A, Alanazi F, et al,. A novel triple suppository of pomegranate, alum and myrrah for fast treatment of hemorrhoids. Saudi patent office , 2015, No ( 4550)
36. N. RAHMANI1, M. KHADEMLOO2, K. VOSOUGHI1, S. ASSADPOUR, Effects of Aloe vera cream on chronic anal fissure pain, wound healing and hemorrhaging upon defection: a prospective double blind clinical trial, European Review for Medical and Pharmacological Sciences, 2014; 18: 1078-1084

## Claims

1. A pharmaceutical composition comprising:
- 5-20% w/w of ethanolic pomegranate peel extract,
- 5-20% w/w of aqueous Myrrh extract,
- 5-20% w/w Alum, and
- a pharmaceutical carrier.

2. The pharmaceutical composition according to claim 1 further comprising ≤ 5% w/w Aloe Vera oil.

3. The pharmaceutical composition according to claim 1, wherein said pharmaceutical carrier is selected, but not limited to, suppository base, ointment base, cream, gel, emulsion or lotion.

4. The pharmaceutical composition according to any one of the preceding claims, for use in the treatment of anal wounds in a subject in need thereof.

5. The pharmaceutical composition for use according to claim 4, wherein the anal wound is hemorrhoids and/or anal fissure.

## Patentansprüche

1. Eine pharmazeutische Zusammensetzung, umfassend:
- 5-20 % w/w ethanolischer Granatapfelschalenextrakt,
- 5-20 % w/w wässriger Myrrhe-Extrakt,
- 5-20 Gew.-% Alaun und
- ein pharmazeutischer Träger.

2. Pharmazeutische Zusammensetzung nach Anspruch 1, ferner umfassend < 5 Gew.-% Aloe Vera-Öl.

3. Pharmazeutische Zusammensetzung nach Anspruch 1, wobei der pharmazeutische Träger ausgewählt, aber nicht beschränkt auf Suppositoriengrundlage, Salbengrundlage, Creme, Gel, Emulsion oder Lotion ist.

4. Pharmazeutische Zusammensetzung nach einem der vorangehenden Ansprüche zur Verwendung bei der Behandlung von Analwunden bei einem Patienten, der dies benötigt.

5. Pharmazeutische Zusammensetzung nach Anspruch 4, wobei die Analwunde Hämorrhoiden und/oder Analfissuren sind.

## Revendications

1. Une composition pharmaceutique comprenant :
- 5-20% p/p d'extrait d'écorce de grenade éthanolique,
- 5- 20% p/p d'extrait de myrrhe aqueux,
- 5- 20% p/p d'alun,
- Un excipient pharmaceutique.

2. La composition pharmaceutique selon la revendication 1 comprenant ≤ 5% p/p d'huile d'Aloe Vera.

3. La composition pharmaceutique selon la revendication 1 où le dit excipient pharmaceutique est sélectionné de, mais non limité à : base de suppositoire, base de pommade, crème, gel, émulsion ou lotion.

4. La composition pharmaceutique selon l'une des revendications précédentes, destinée à être utilisée dans le traitement des plaies anales chez un sujet en ayant besoin.

5. La composition pharmaceutique pour utilisation selon la revendication 4, dans laquelle la plaie anale est une hémorroïde et/ou une fissure anale.
